# EUROPÄISCHE PATENTSCHRIFT

(11) **EP 1 095 269 B1**
(45) Veröffentlichungstag und Bekanntmachung des Hinweises auf die Patenterteilung: **02.06.2004**
(21) Anmeldenummer: 99932860.2
(22) Anmeldetag: 09.07.1999
(51) Int. Cl.: G01N 33/497, A61B 5/083

(54) **VORRICHTUNG ZUR BESTIMMUNG DES KOHLENDIOXIDGEHALTES IN AUSGEATMETER ATEMLUFT**
DEVICE FOR DETERMINING THE CONTENT OF CARBON DIOXIDE IN EXHALED AIR
DISPOSITIF POUR DETERMINER LA TENEUR EN DIOXYDE DE CARBONE DANS DE L'AIR EXPIRE

(30) Priorität: 10.07.1998 DE 19831022
(43) Veröffentlichungstag der Anmeldung: 02.05.2001
(73) Patentinhaber: Stemple Ing. Büro, 86916 Kaufering (DE)
(72) Erfinder: STEMPLE, Günter, D-86916 Kaufering (DE)
(74) Vertreter: Nöth, Heinz, Dipl.-Phys.
(86) Internationale Anmeldenummer: PCT/EP1999/004840
(87) Internationale Veröffentlichungsnummer: WO 2000/003243

(56) Entgegenhaltungen:
- WO-A-89/03523
- US-A- 5 046 491
- US-A- 5 400 781
- US-A- 5 474 060

## Beschreibung

Die Erfindung betrifft ein Verfahren nach dem Oberbegriff des Patentanspruches 1 und eine Vorrichtung nach dem Oberbegriff des Patentanspruches 2.

Aus der WO 89/03523 sind ein Verfahren und eine Vorrichtung bekannt, bei denen in der Ausatemluft mittels eines insbesondere als Infrarotanalysators ausgebildeten Sensors der Kohlendioxidgehalt der Ausatemluft bestimmt und aufgezeichnet wird. Dabei werden in einem Hauptstrom die gesamte Ausatemluft durch einen Analysekanal geleitet und mit dem Sensor Messsignale erzeugt, die dem Kohlendioxidgehalt der im Analysekanal befindlichen Ausatemluft proportional sind. An den Sensor ist eine Auswerteeinrichtung mit Anzeigemitteln angeschlossen, welche die Messsignale auswertet und anzeigt. Die Ausatemluft wird im Analysekanal über eine Luftleitung zugeführt. Für den Transport der Ausatemluft im Analysekanal sorgt eine Vakuumpumpe, die an den Analysekanal angeschlossen ist.

Aus der US-A 5,474,060 ist eine Vorrichtung zur Bestimmung des Kohlendioxidgehaltes von Ausatemluft bekannt, bei welcher eine Maske verwendet wird, in deren Maskeninnenraum Mund und Nase aufgenommen werden. In einem nach unten gerichteten Ansatzstück der Maske ist eine Sonde angeordnet, über welche dem Maskeninnenraum Sauerstoff zuführbar ist. Ferner ist mittels eines Adapters eine Luftleitung an die Maske anschließbar, um über die Luftleitung Ausatemluft einer Messvorrichtung für den Kohlendioxidgehalt zuzuleiten.

Aus der US-A 5,046,491 ist eine Vorrichtung zur Bestimmung des Kohlendioxidgehaltes in Ausatemluft bekannt, bei welcher Luftleitungen an ihren einen Ende in Nasenhöhlungen einsetzbar sind und die über eine Luftleitung an ein Gasanalysegerät angeschlossen sind.

Beim bekannten Verfahren und bei den bekannten Vorrichtungen sind relativ lange Luftführungswege für die Ausatemluft erforderlich. Hieraus ergibt sich ein gegen Verschmutzung und Verschluss anfälliges relativ großes Totvolumen zwischen den Nasenhöhlungen des Patienten oder dem Maskeninnenraum und dem Sensor, dem die Ausatemluft für die Analyse im Hinblick auf den CO₂-Gehalt zugeführt wird.

Aufgabe der Erfindung ist es, ein Verfahren und eine Vorrichtung der eingangs genannten Art zu schaffen, bei welchen mit geringem Aufwand genaue Messergebnisse erzielt werden.

Diese Aufgabe wird beim Verfahren erfindungsgemäß durch die kennzeichnenden Merkmale des Patentanspruches 1 und bei der Vorrichtung durch die kennzeichnenden Merkmale des Patentanspruches 2 gelöst.

Dem Maskeninnenraum kann über eine Sonde Sauerstoff zugeführt werden. In der Sonde kann die Öffnung für überschüssigen Sauerstoff vorgesehen sein, so dass die Messergebnisse nicht beeinträchtigt werden. Ferner können in der Maske Öffnungen für einen Gasaustausch zwischen dem Maskeninnenraum und der Außenluft vorgesehen sein.

Mit Hilfe der Maske kann sowohl nasale als auch orale Ausatemluft jeweils einzeln oder gemeinsam für die Messung erfasst werden.

Der Sensor und die dem Sensor zugeführten und in die Nasenlöcher einsetzbaren Luftleitungen können an einem an der Nase des Patienten fixierbaren Adapter befestigt sein.

In bevorzugter Weise ist der Sensor mittels des Adapters über dem Nasenrücken fixierbar, wobei die beiden Luftleitungen einem gemeinsamen Analysekanal für die Durchführung der CO₂-Analyse mittels des Sensors zugeführt werden. Der Querschnitt des Analysekanals entspricht in bevorzugter Weise etwa der Summe der beiden Querschnitte der Luftleitungen. In bevorzugter Weise sind die Durchmesser der beiden Luftleitungen wesentlich geringer als die Durchmesser der beiden Nasenöffnungen, in welche die Leitungsenden der Luftleitungen eingesetzt sind. Auch ist das Gesamtvolumen, welches sich aus den Volumina der beiden Luftleitungen und des Analysekanals des Sensors zusammensetzt, bedeutend geringer als die beiden Volumina der Nasenhöhlen.

Durch diese Anordnung läßt sich für das Nebenstromverfahren aufgrund der äußerst kurzen Übertragungsstrecke der Ausatemluft von den Nasenöffnungen zum Sensor eine hohe Meßgenauigkeit erreichen wie beim Hauptstromverfahren. Aufgrund des geringen Totvolumens in den Luftleitungen, welche bevorzugt vor dem Sensor zusammengeführt sind, wird die Anordnung unempfindlich gegen Verschmutzung und Verschluß. Zur Reinigung der Luftleitungen sind Wasserfilter und Wasserfallen und -pumpen zum Beseitigen von Verschmutzung und Verschlüssen nicht erforderlich. Es ist daher mit einem geringen Aufwand eine störungsfreie Messung möglich. Die Verbindung zwischen dem Sensor und dem Auswerte- und Anzeigegerät erfolgt mit Hilfe eines elektrischen Kabels, welches die vom Sensor gelieferten Meßsignale überträgt. Diese Übertragungsstrecke ist beliebig wählbar. Hieraus ergibt sich eine einfache und übersichtliche Durchführung des Meßvorgangs, insbesondere bei der Nebenstromanwendung bei der Kapnographie.

Am Adapter kann eine Aufnahmeeinrichtung vorgesehen sein, in die der Sensor lösbar einsetzbar ist. Hierdurch ist es möglich, sowohl für das Hauptstromverfahren als auch für das Nebenstromverfahren einen und denselben Sensor, der mit dem Auswerte- und Anzeigegerät verbunden bzw. verbindbar ist, zu verwenden.

Für den Adapter können zwei gelenkig miteinander verbundene und zu beiden Seiten des Nasenrückens an der Nase fixierbare Fixierschenkel vorgesehen sein. Die Fixierschenkel können in bevorzugter Weise mit Klebeflächen versehen sein, so daß der Adapter auf der Nasenhaut nach Art eines Pflasters (z.B. Schnarchpflasters) aufklebbar ist. lm aufgeklebten Zustand können die Fixierschenkel unter einer Vorspannung stehen, durch weiche auf die Nasenflügel bzw. Nasenöffnungswände eine erweiternde Vorspannung ausgeübt wird. Hierdurch wird die Atmung erleichtert.

Ferner kann wenigstens eine der beiden Luftleitungen so ausgebildet sein, daß eine Sonde, insbesondere O₂-Sonde, in die Luftleitung derart eingesetzt werden kann, daß die Ausatemluftzufuhr zum Sensor unterbrochen wird. Hierzu kann die Sonde fluchtend mit dem in die Nasenöffnung eingesetzten im wesentlichen geradlinig verlaufenden Leitungsende in die Luftleitung eingesetzt werden. Es ist auch möglich, daß die eingesetzte Sonde ganz oder teilweise das in die Nasenöffnung eingesetzte Leitungsende durchsetzt. Hierzu kann eine entsprechende Perforation an der Luftleitung, weiche insbesondere aus flexiblem Material (Schlauchmaterial) besteht, vorgesehen sein. Hierbei kann Sauerstoff über die Sonde in eine der beiden Nasenöffnungen eingeleitet werden, während über die andere Nasenöffnung die CO₂-Messung der Ausatemluft weiterhin erfolgt.

Anhand der Figuren wird an einem Ausführungsbeispiel die Erfindung noch näher erläutert. Es zeigt:
- Fig. 1: ein Ausführungsbeispiel der Erfindung aus einer in die Nasenöffnungen gerichteten Blickrichtung;
- Fig. 2: das Ausführungsbeispiel der Fig. 1 in Draufsicht;
- Fig. 3: in perspektivischer Darstellung eine mit A in Fig. 1 bezeichnete Einzelheit an einem Leitungsende einer Luftleitung; und
- Fig. 4: ein weiteres Ausführungsbeispiel.

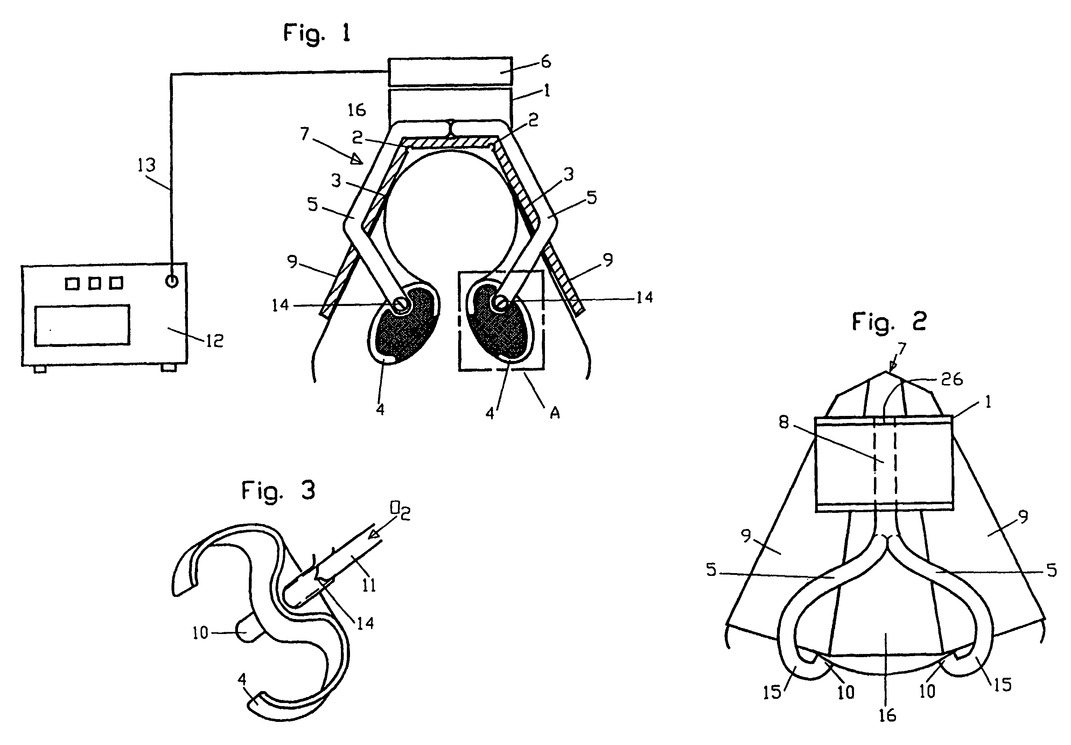

Das in den Fig. 1 bis 3 dargestellte Ausführungsbeispiel besitzt einen über ein elektrisches Kabel 13 an eine Auswerte- und Anzeigeeinrichtung 12 angeschlossenen Sensor 6. Der Sensor 6 kann in eine Aufnahmeeinrichtung 1, welche an einem Adapter 7 befestigt ist, eingesetzt werden. Am Adapter 7 ist neben der Aufnahmeeinrichtung 1 für den Sensor 6 eine Luftzufuhr zu einem Analysekanal 8, welcher in der Haltevorrichtung 1 vorgeformt sein kann, ferner befestigt. Die Luftzufuhr besteht aus zwei Luftleitungen 5, welche mit ihren Leitungsenden 10 in Nasenöffnungen eingesetzt werden können. An dem in die Nasenöffnungen einsetzbaren Leitungsenden 10 besitzen die Luftleitungen 5 Halteeinrichtungen 4, welche in ihrer Form an die Nasenöffnungen angepaßt sind. Die Halteeinrichtungen 4 besitzen die Form von Paßstücken, die im wesentlichen formschlüssig in die Nasenöffnungen eingesetzt werden können. Durch die Halteeinrichtungen 4 in Form von offenen Paßringen wird gewährleistet, daß die in die Nasenöffnungen eingesetzten Leitungsenden 10 in einem Bereich in der Nähe der Nasenspitze gehalten werden. Hierdurch können eventuell vorhandene Sekrete in der Nase des liegenden Patienten ungehindert austreten, ohne die Luftleitungsenden 10 zu verschließen. Die Halteeinrichtungen 4 können in Form von elastischen Spreizklammern ausgebildet sein. Hierdurch erreicht man eine Aufweitung der Nasenöffnung. Da die Einlaßöffnungen an den Leitungsenden 10 wesentlich geringer sind als die Querschnitte der Nasenöffnungen wird die Atmung kaum beeinträchtigt.

Die in die Nasenöffnungen eingeführten Leitungsenden 10 verlaufen im wesentlichen geradlinig. Hieran schließen sich gekrümmte Leitungsteile 15 an. Diese gehen in zwei im wesentlichen in V- oder U-Form aufeinanderzu verlaufende Leitungsteile über. Vor dem Einlaß in die Haltevorrichtung bzw. in den Analysekanal 8 des Sensors 6 werden die beiden Luftleitungen 5, wie es insbesondere aus der Fig. 2 zu ersehen ist, zusammengeführt. An seinem anderen Ende 26 ist der Analysekanal 8 offen.

Der Sensor kann in bekannter Weise ausgebildet sein, wobei ein miniaturisiertes System verwendet werden kann. Die Luftleitungen 5 bestehen in bevorzugter Weise aus flexiblen Schläuchen.

Der Adapter besitzt zwei gelenkig miteinander verbundene Fixierschenkel 9. Die Fixierschenkel 9 besitzen beim dargestellten Ausführungsbeispiel in Form von Klebestreifen ausgebildete Klebeflächen 3. Der Adapter 7 besitzt ferner einen zwischen den beiden Fixierschenkeln 9 liegenden Verbindungsstreifen 16. An diesem Verbindungsstreifen 16 kann die Aufnahmeeinrichtung 1 für den einsteckbaren Sensor 6 befestigt sein. Die beiden Fixierschenkel 9 sind über bevorzugt federnde Gelenke 2 mit dem Verbindungsstreifen 16 verbunden. Wenn der Adapter 7 auf die Nasenaußenfläche aufgeklebt ist, liegt die Halteeinrichtung 1 über dem Nasenrücken. Aufgrund der Federwirkung der beiden Gelenke 2 werden die beiden Fixierschenkel 9 nach außen gedrückt, so daß die Nasenflügel und damit die Nasenhöhlungen für eine bessere Atmung ausgeweitet werden. Durch die flexible Ausbildung der Luftleitungen 5 und des Adapters 7 erreicht man eine hohen Anpassungsgrad an verschiedene Nasengrößen, so daß nur eine geringe Anzahl an verschiedene Adaptergrößen in Bereitschaft gehalten werden müssen.

Wie insbesondere aus der Fig. 3 zu ersehen ist, kann eine am Ende des gekrümmten Leitungsstückes 15 vorgesehene Perforierung 14 in beiden oder in wenigstens einem der beiden Luftleitungen 5 von einer Sonde 11 durchstoßen werden. Beim Einsetzen der Sonde 11 in einen der beiden Luftleitungen 5 fluchtet die Sonde 11 in ihrer axialen Richtung mit der axialen Richtung des jeweiligen Leitungsendes 10, das in die Nasenöffnung eingesetzt ist wie es aus Fig. 3 zu ersehen ist. Dabei wird die Zufuhr von Ausatemluft zum Sensor 6 hin unterbrochen. Auf diese Weise kann Sauerstoff durch die Sonde 11 zugeführt werden, wobei gleichzeitig die CO₂-Messung der über die andere Luftleitung 5 dem Sensor zugeführten Ausatemluft durchgeführt werden kann.

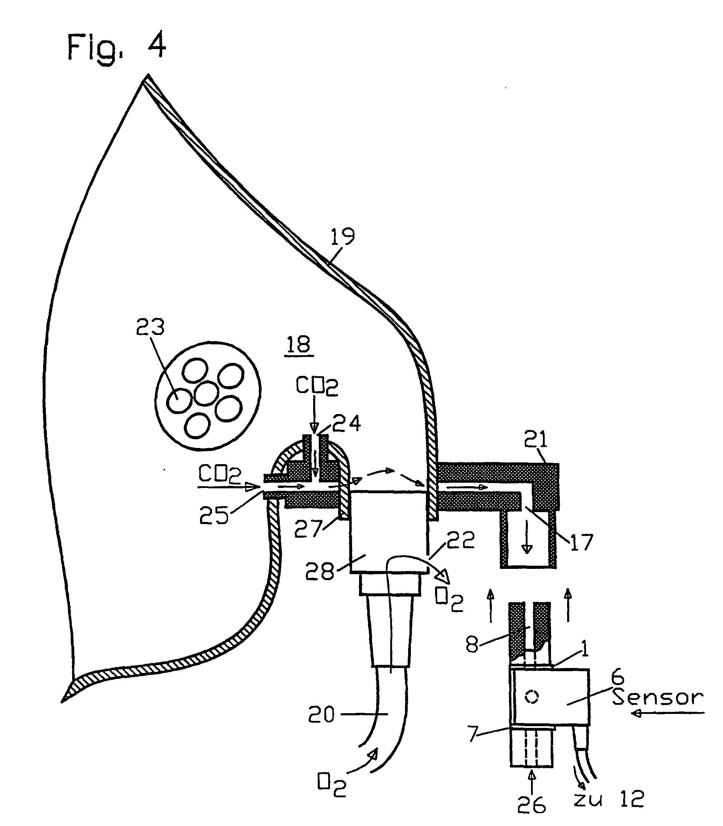

Bei dem in der Fig. 4 dargestellten Ausführungsbeispiel ist eine über Mund und Nase aufsetzbare Maske 19 vorgesehen. Eine zum Analysekanal 8 geführte Luftleitung 7 befindet sich in einem Maskenadapter 21. Der Maskenadapter 21 ist mit einer Ringöffnung über ein nach unten gerichtetes Ansatzstück 27 der Maske 19 geschoben. In das Ansatzstück 27 kann eine Sonde 20 für Sauerstoffzufuhr eingesetzt sein.

Die Luftleitung 17 ist um das Ansatzstück 27 im Maskenadapter 21 geführt und mit zwei Eintrittsöffnungen 24 und 25 verbunden, welche in der Maske 19 vorgesehen sind. Über die Eintrittsöffnungen 24 und 25 wird eine Strömungsverbindung zwischen einem Maskeninnenraum 18 und der Luftleitung 17 hergestellt. Die Eintrittsöffnung 24 ist bei aufgesetzter Maske 19 mit den Nasenöffnungen des Patienten ausgerichtet und die Eintrittsöffnung 25 ist etwa mit dem Mund des Patienten ausgerichtet. Von den Eintrittsöffnungen 24 und 25 können separate schlauchförmige Verbindungen zu Mund und Nase geführt sein.

An den beiden Seitenwänden der Maske 19 sind ferner Öffnungen 23 vorgesehen, welche einen Gasaustausch zwischen dem Maskeninnenraum 18 und der Außenluft ermöglichen.

Auf den Maskenadapter 21 ist der Sensoradapter 7, an dessen Aufnahmeeinrichtung 1 der Sensor 6 lösbar aufgesteckt ist, aufschiebbar, so daß die Luftleitung 17 in den Analysekanal 8 mündet. Die Verbindung zwischen der Luftleitung 17 und dem Analysekanal 8 erfolgt am einen Ende des Analysekanals 8. An seinem anderen Ende 26 ist der Analysekanal 8 geöffnet und mündet in die Außenluft.

In der Sonde 20 ist eine Öffnung 22 im Bereich ihres Verbindungsstückes 28 mit der Maske 19 vorgesehen. Die Öffnung 22 ist nach außen geöffnet und gewährleistet einen Austritt an überschüssigem Sauerstoff an die Außenluft. Die Austrittsöffnung 22 befindet sich hierzu unterhalb der Kante des Ansatzstückes 27, in welches das Verbindungsstück 28 der Sonde 20 in die Maske 19 eingesetzt ist.

Beim Ausatmen hat die Ausatemluft einen die Eigenströmung bewirkenden Druck, durch welchen überschüssiger Sauerstoff durch die Öffnung 22 nach außen verdrängt wird. Der in den Maskeninnenraum 18 über die Sonde 20 gelieferte Sauerstoff kann mit einem entsprechenden Druck beaufschlagt sein, daß keine Beeinträchtigung der CO₂ - Messung in der Ausatemluft sich ergibt. Durch die Eintrittsöffnungen 24 und 25 gelangt genügend Ausatemluft in die Luftleitung 17 für die im Analysekanal 8 durchzuführende Messung. Durch entsprechende Verbindungen zwischen der jeweiligen Eintrittsöffnung 24 und 25 mit Nase und Mund kann auch eine separate nasale oder orale Erfassung der Ausatemluft durchgeführt werden.

### Bezugszeichenliste

- 1: Aufnahmeeinrichtung
- 2: Gelenk
- 3: Klebeflächen
- 4: Halteeinrichtungen
- 5: Luftleitung
- 6: Sensor
- 7: Adapter
- 8: Analysekanal
- 9: Fixierschenkel
- 10: Leitungsende
- 11: Sonde
- 12: Auswerte- und Anzeigeeinrichtung
- 13: elektrisches Kabel
- 14: Perforierung
- 15: Leitungsstück
- 16: Verbindungsstreifen
- 17: Luftleitung
- 18: Maskeninnenraum
- 19: Maske
- 20: Sonde
- 21: Maskenadapter
- 22: Öffnung
- 23: Öffnung
- 24: Eintrittsöffnung
- 25: Eintrittsöffnung
- 26: offenes Ende
- 27: Ansatzstück
- 28: Verbindungsstück

## Patentansprüche

1. Verfahren zur Bestimmung des Kohlendioxid (CO₂)-Gehaltes in Ausatemluft, bei dem die Ausatemluft über eine Luftleitung in einem Analysekanal einem Sensor zur Erzeugung von dem Kohlendioxid-Gehalt proportionalen Messsignalen zugeführt wird, wobei bei der Durchführung der Messung der Analysenkanal an seinem einen Ende an die Luftleitung angeschlossen wird und von einer an den Sensor angeschlossenen Auswerteeinrichtung mit Anzeigemitteln die Messsignale ausgewertet und angezeigt werden,
**dadurch gekennzeichnet, dass** die Luftleitung und der Analysekanal mit einem derart geringen Totvolumen vorgesehen werden, dass die vom Sensor zu messende Ausatemluft nur mit dem die Eigenströmung bewirkenden Druck dem Analysekanal zugeführt wird und der Analysekanal an seinem anderen Ende gegenüber der Außenluft offengehalten wird.

2. Vorrichtung zur Bestimmung des Kohlendioxid (CO₂)-Gehaltes in Ausatemluft mit
- einem Analysekanal (8);
- einer Luftleitung (17), welche für die Zufuhr von zu messender Ausatemluft in den Analysekanal (8) mit einem Ende des Analysekanals (8) verbunden ist;
- einem Sensor (6), welcher dem Kohlendioxidgehalt der im Analysekanal (8) befindlichen Ausatemluft proportionale Messsignale erzeugt; und
- einer an den Sensor (6) angeschlossenen Auswerte- und Anzeigeeinrichtung (12) für die Messsignale;
**dadurch gekennzeichnet, dass**
- der Analysekanal (8) am anderen Ende gegenüber der Außenluft offen ist;
- die Luftleitung (17) und der Analysekanal (8) zwischen einer Mund und Nase umschließenden Maske (19) oder in Nasenöffnungen eingesetzten Luftleitungen (5) und dem Sensor (6) ein derart geringes Totvolumen aufweisen, dass
- in der Luftleitung (17) oder den Luftleitungen (5) und dem Analysekanal (8) nur der Druck, welcher durch die Eigenströmung der vom Sensor (6) gemessenen Ausatemluft erzeugt ist, vorhanden ist.

3. Vorrichtung nach Ansprüche 2,
**dadurch gekennzeichnet, dass** an der Maske (19) ein Maskenadapter (21), welcher die die Ausatemluft führende Luftleitung (17) aufnimmt, auswechselbar befestigt ist.

4. Vorrichtung nach Anspruch 2 und 3,
**dadurch gekennzeichnet, dass** der Analysekanal (8) in einem Sensoradapter (7) angeordnet ist, welcher lösbar mit dem Maskenadapter (21) verbunden ist.

5. Vorrichtung nach einem der Ansprüche 2 bis 4,
**dadurch gekennzeichnet, dass** dem Maskeninnenraum (18) über eine Sonde (20) Sauerstoff zuführbar ist.

6. Vorrichtung nach Anspruch 5,
**dadurch gekennzeichnet, dass** in der Sonde (20) eine Öffnung (22) für überschüssigen Sauerstoff vorgesehen ist.

7. Vorrichtung nach einem der Ansprüche 2 bis 6,
**dadurch gekennzeichnet, dass** in der Maske (19) Öffnungen (23) für einen Gasaustausch zwischen dem Maskeninnenraum (18) und der Außenluft vorgesehen sind.

8. Vorrichtung nach Anspruch 2,
**dadurch gekennzeichnet, dass** der Sensor (6) und die Luftleitungen (5) an einem an der Nase fixierbaren Sensoradapter (7) befestigbar oder befestigt sind.

9. Vorrichtung nach Anspruch 8,
**dadurch gekennzeichnet, dass** der Sensor (6) mittels des Sensoradapters (7) über dem Nasenrücken fixierbar ist.

10. Vorrichtung nach Anspruch 8 oder 9,
**dadurch gekennzeichnet, dass** die beiden Luftleitungen (5) vor dem Sensor (6) zusammengeführt sind.

11. Vorrichtung nach einem der Ansprüche 8 bis 10,
**dadurch gekennzeichnet, dass** der Sensoradapter (7) eine Aufnahmeeinrichtung (1) für den Sensor (6) aufweist.

12. Vorrichtung nach Anspruch 11,
**dadurch gekennzeichnet, dass** der Sensor (6) in die Aufnahmeeinrichtung (1) lösbar einsetzbar ist.

13. Vorrichtung nach einem der Ansprüche 8 bis 10,
**dadurch gekennzeichnet, dass** der Sensoradapter (7) zwei gelenkig verbundene und zu beiden Seiten des Nasenrückens auf der Nase fixierbare Fixierschenkel (9) aufweist.

14. Vorrichtung nach einem der Ansprüche 8 bis 13,
**dadurch gekennzeichnet, dass** der Sensoradapter (7) auf die Nasenhaut aufklebbar ist.

15. Vorrichtung nach Anspruch 13 und 14,
**dadurch gekennzeichnet, dass** bei auf die Nasenhaut aufgeklebten Fixierschenkeln (9) die Fixierschenkel unter einer die Nasenhöhlungen erweiternden Vorspannung stehen.

16. Vorrichtung nach einem der Ansprüche 8 bis 10,
**dadurch gekennzeichnet, dass** die Luftleitungen (5) aus flexiblem Material bestehen.

17. Vorrichtung nach einem der Ansprüche 8 bis 16,
**dadurch gekennzeichnet, dass** das jeweilige in eine Nasenöffnung eingesetzte Leitungsende (10) der Luftleitung (5) mittels einer Halteeinrichtung (4), die an die Nasenöffnung angepasst ist, in der Nasenöffnung fixierbar ist.

18. Vorrichtung nach Anspruch 17,
**dadurch gekennzeichnet, dass** das jeweilige Leitungsende (10) an der Halteeinrichtung (4) derart befestigt ist, dass es in einem der Nasenspitzen benachbarten Bereich der Nasenöffnung gehalten ist.

19. Vorrichtung nach einem der Ansprüche 2 bis 11,
**dadurch gekennzeichnet, dass** in wenigstens eine der Luftleitungen (5) eine Sonde (11) von außen einsetzbar ist derart, dass die Zufuhr der Ausatemluft zum Sensor (6) unterbrochen ist.

20. Vorrichtung nach Anspruch 19,
**dadurch gekennzeichnet, dass** die Sonde (11) mit dem in die Nasenhöhlung eingesetzten Leitungsende (10) fluchtet oder in dieses wenigstens zum Teil eingesetzt ist.

21. Vorrichtung nach Anspruch 19 oder 20,
**dadurch gekennzeichnet, dass** die Sonde (11) eine Sauerstoffsonde ist.

## Claims

1. A method of determining the carbon dioxide (CO₂) content in exhaled air, in which the exhaled air is fed by way of an air conduit in an analysis passage to a sensor for producing measurement signals which are proportional to the carbon dioxide content, wherein when performing the measurement operation the analysis passage is connected at its one end to the air conduit and the measurement signals are evaluated and displayed by an evaluation device which is connected to the sensor and which has display means, **characterised in that** the air conduit and the analysis passage are provided with such a small dead volume that the exhaled air to be measured by the sensor is passed to the analysis passage only at the pressure causing the inherent flow and the analysis passage is kept open at its other end in relation to the outside air.

2. Apparatus for determining the carbon dioxide (CO₂) content in exhaled air, comprising
- an analysis passage (8);
- an air conduit (17) which is connected to one end of the analysis passage (8) for the feed of exhaled air to be measured into the analysis passage (8);
- a sensor (6) which produces measurement signals proportional to the carbon dioxide content of the exhaled air which is in the analysis passage (8); and
- an evaluation and display device (12) which is connected to the sensor (6), for the measurement signals;
**characterised in that**
- the analysis passage (8) is open in relation to the outside air at the other end;
- the air conduit (17) and the analysis passage (8) between a mask (19) enclosing mouth and nose or air conduits (5) inserted into nostrils and the sensor (6), are of such a small dead volume that
- only the pressure which is produced by the inherent flow of the exhaled air measured by the sensor (6) obtains in the air conduit (17) or the air conduits (5) and the analysis passage (8).

3. Apparatus according to claim 2 **characterised in that** a mask adaptor (21) which receives the air conduit (17) carrying the exhaled air is replaceably secured to the mask (19).

4. Apparatus according to claim 2 and claim 3 **characterised in that** the analysis passage (8) is arranged in a sensor adaptor (7) which is releasably connected to the mask adaptor (21).

5. Apparatus according to one of claims 2 to 4 **characterised in that** oxygen can be fed to the mask interior (18) by way of a probe (20).

6. Apparatus according to claim 5 **characterised in that** provided in the probe (20) is an opening (22) for excess oxygen.

7. Apparatus according to one of claims 2 to 6 **characterised in that** provided in the mask (19) are openings (23) for gas exchange between the mask interior (18) and the outside air.

8. Apparatus according to claim 2 **characterised in that** the sensor (6) and the air conduits (5) are secured or can be secured to a sensor adaptor (7) which can be fixed to the nose.

9. Apparatus according to claim 8 **characterised in that** the sensor (6) can be fixed by means of the sensor adaptor (7) over the bridge of the nose.

10. Apparatus according to claim 8 or claim 9 **characterised in that** the two air conduits (5) are brought together upstream of the sensor (6).

11. Apparatus according to one of claims 8 to 10 **characterised in that** the sensor adaptor (7) has a receiving device (1) for the sensor (6).

12. Apparatus according to claim 11 **characterised in that** the sensor (6) can be fitted releasably into the receiving device (1).

13. Apparatus according to one of claims 8 to 10 **characterised in that** the sensor adaptor (7) has two hingedly connected fixing limbs (9) which can be fixed on the nose at both sides of the bridge of the nose.

14. Apparatus according to one of claims 8 to 13 **characterised in that** the sensor adaptor (7) can be stuck on the skin of the nose.

15. Apparatus according to claim 13 and claim 14 **characterised in that** when fixing limbs (9) are stuck on the skin of the nose the fixing limbs are under a prestress which enlarges the nasal cavities.

16. Apparatus according to one of claims 8 to 10 **characterised in that** the air conduits (5) comprise flexible material.

17. Apparatus according to one of claims 8 to 16 **characterised in that** the respective end (10) of the air conduit (5), which is fitted into a nostril, can be fixed in the nostril by means of a holding device (4) which is adapted to the nostril.

18. Apparatus according to claim 17 **characterised in that** the respective end (10) of the conduit is secured to the holding device (4) in such a way that it is held in a region of the nostril, which is adjacent to the tip of the nose.

19. Apparatus according to one of claims 2 to 11 **characterised in that** a probe (11) can be inserted from the outside into at least one of the air conduits (5) in such a way that the supply of exhaled air to the sensor (6) is interrupted.

20. Apparatus according to claim 19 **characterised in that** the probe (11) aligns with the conduit end (10) inserted into the nasal cavity or is at least partially fitted into said conduit end.

21. Apparatus according to claim 19 or claim 20 **characterised in that** the probe (11) is an oxygen probe.

## Revendications

1. Procédé pour déterminer la teneur en dioxyde de carbone (CO₂) dans de l'air expiré, dans lequel l'air expiré est envoyé par l'intermédiaire d'une conduite d'air dans un canal d'analyse à un capteur pour générer des signaux de mesure proportionnels à la teneur en dioxyde de carbone, le canal d'analyse étant raccordé à l'une de ses extrémités à la conduite d'air lors de la réalisation de la mesure et les signaux de mesure étant analysés et affichés par un dispositif d'analyse avec moyens d'affichage raccordé au capteur, **caractérisé en ce que** la conduite d'air et le canal d'analyse sont prévus avec un volume mort si faible que l'air expiré à mesurer par le capteur n'est envoyé au canal d'analyse qu'avec la pression provoquant l'écoulement propre et le canal d'analyse est maintenu ouvert par rapport à l'air extérieur à son autre extrémité.

2. Dispositif pour déterminer la teneur en dioxyde de carbone (CO₂) dans de l'air expiré comprenant
- un canal d'analyse (8) ;
- une conduite d'air (17) reliée à une extrémité du canal d'analyse (8) pour l'arrivée d'air expiré à mesurer dans le canal d'analyse (8) ;
- un capteur (6) qui génère des signaux de mesure proportionnels à la teneur en dioxyde de carbone de l'air expiré se trouvant dans le canal d'analyse (8) ; et
- un dispositif d'analyse et d'affichage (12) pour les signaux de mesure raccordé au capteur (6) ;
**caractérisé en ce que**
- le canal d'analyse (8) est ouvert à l'autre extrémité par rapport à l'air extérieur ;
- la conduite d'air (17) et le canal d'analyse (8) présentent entre un masque (19) enfermant la bouche et le nez ou des conduites d'air (5) logées dans des narines et le capteur (6) un volume mort si faible que
- seule la pression produite par l'écoulement propre de l'air expiré mesuré par le capteur (6) est présente dans la conduite d'air (17) ou dans les conduites d'air (5) et dans le canal d'analyse (8).

3. Dispositif suivant la revendication 2, **caractérisé en ce qu'**un adaptateur de masque (21) qui reçoit la conduite d'air (17) guidant l'air expiré est fixé de façon interchangeable sur le masque (19).

4. Dispositif suivant les revendications 2 et 3, **caractérisé en ce que** le canal d'analyse (8) est disposé dans un adaptateur de capteur (7) qui est relié de façon amovible à l'adaptateur de masque (21).

5. Dispositif suivant l'une des revendications 2 à 4, **caractérisé en ce que** de l'oxygène peut être envoyé dans l'espace intérieur du masque (18) par l'intermédiaire d'une sonde (20).

6. Dispositif suivant la revendication 5, **caractérisé en ce qu'** une ouverture (22) est prévue dans la sonde (20) pour de l'oxygène excédentaire.

7. Dispositif suivant l'une des revendications 2 à 6, **caractérisé en ce que** des ouvertures (23) sont prévues dans le masque (19) pour un échange gazeux entre l'espace intérieur du masque (18) et l'air extérieur.

8. Dispositif suivant la revendication 2, **caractérisé en ce que** le capteur (6) et les conduites d'air (5) peuvent être attachés ou sont attachés sur un adaptateur de capteur (7) pouvant être fixé sur le nez.

9. Dispositif suivant la revendication 8, **caractérisé en ce que** le capteur (6) peut être fixé sur l'arête du nez au moyen de l'adaptateur de capteur (7).

10. Dispositif suivant l'une des revendications 8 et 9, **caractérisé en ce que** les deux conduites d'air (5) sont réunies en amont du capteur (6).

11. Dispositif suivant l'une des revendications 8 à 10, **caractérisé en ce que** l'adaptateur de capteur (7) présente un agencement de réception (1) pour le capteur (6).

12. Dispositif suivant la revendication 11, **caractérisé en ce que** le capteur (6) peut être logé de façon amovible dans l'agencement de réception (1).

13. Dispositif suivant l'une des revendications 8 à 10, **caractérisé en ce que** l'adaptateur de capteur (7) présente deux branches de fixation (9) articulées entre elles et pouvant être fixées sur le nez de part et d'autre de l'arête du nez.

14. Dispositif suivant l'une des revendications 8 à 13, **caractérisé en ce que** l'adaptateur de capteur (7) peut être collé sur la peau du nez.

15. Dispositif suivant les revendications 13 et 14, **caractérisé en ce que,** lorsque les branches de fixation (9) sont collées sur la peau du nez, les branches de fixation sont soumises à une précontrainte élargissant les fosses nasales.

16. Dispositif suivant l'une des revendications 8 à 10, **caractérisé en ce que** les conduites d'air (5) sont constituées de matériau flexible.

17. Dispositif suivant l'une des revendications 8 à 16, **caractérisé en ce que** chaque extrémité de conduite (10), logée dans une narine, de la conduite d'air (5) peut être fixée dans la narine au moyen d'un dispositif de retenue (4) adapté à la narine.

18. Dispositif suivant la revendication 17, **caractérisé en ce que** chaque extrémité de conduite (10) est fixée sur le dispositif de retenue (4) de telle sorte qu'elle est maintenue dans une zone de la narine voisine du bout du nez.

19. Dispositif suivant l'une des revendications 2 à 11, **caractérisé en ce qu**'une sonde (11) peut être mise en place de l'extérieur dans au moins l'une des conduites d'air (5) de telle sorte que l'arrivée d'air expiré au capteur (6) soit interrompue.

20. Dispositif suivant la revendication 19, **caractérisé en ce que** la sonde (11) est alignée avec l'extrémité de conduite (10) logée dans la fosse nasale ou. est logée au moins en partie dans cette extrémité.

21. Dispositif suivant l'une des revendications 19 et 20, **caractérisé en ce que** la sonde (11) est une sonde à oxygène.
